(19) Europäisches Patentamt
European Patent Office
Office européen des brevets

(11) Veröffentlichungsnummer: **0 001 982**
**B1**

(12)

# EUROPÄISCHE PATENTSCHRIFT

(51) Int. Cl.³: C 07 C 85/11, C 07 C 89/00, C 07 C 143/66, C 07 C 143/64, C 07 C 91/44, C 07 C 91/46, C 07 C 101/74, C 07 C 101/76

(21) Anmeldenummer: **78101177.0**

(22) Anmeldetag: **19.10.78**

(54) **Verfahren zur Herstellung aromatischer Aminohydroxyverbindungen**

(30) Priorität: **16.11.77 DE 2751136**

(43) Veröffentlichungstag der Anmeldung:
**30.05.79 Patentblatt 79/11**

(45) Bekanntmachung des Hinweises auf die Patenterteilung:
**10.12.80 Patentblatt 80/25**

(84) Benannte Vertragsstaaten:
**BE CH DE FR GB**

(56) Entgegenhaltungen:
**HOUBEN-WEYL "Methoden der organischen Chemie"**
**4. Auflage, Band XI, Teil 1, 1957,**
**Georg Thieme Verlag, Stuttgart,**
**Seiten 452 bis 457 und 490 bis 494**

(73) Patentinhaber: **BAYER AG**
**Zentralbereich Patente, Marken und Lizenzen**
**D-5090 Leverkusen 1, Bayerwerk (DE)**

(72) Erfinder: **Pfister, Theodor, Dr.**
**Siller Strasse 51**
**D-5600 Wuppertal-1 (DE)**

**EP 0 001 982 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Verfahren zur Herstellung aromatischer Aminohydroxyverbindungen

Die Erfindung betrifft ein Verfahren zur Herstellung aromatischer Aminohydroxyverbindungen durch Reduktion von entsprechenden aromatischen Nitrosohydroxyverbindungen mit Hydrazin.

Es ist bekannt, 2 - Nitroso - 5 - acetyl - amino - 1 - naphthol mit Phenylhydrazin zu 2 - Amino - 5 - acetyl - amino - 1 - naphthol ohne Zusatz eines Katalysators zu reduzieren (Houben-Weyl, Methoden der organischen Chemie, Band XI/1, Seite 494 (1957)).

Es ist außerdem bekannt, aromatische Nitrosoverbindungen, wie p-Nitrosodiethylanilin, mit Hydrazin in Gegenwart von Raney-Nickel als Katalysator und Ethylalkohol als Lösungsmittel zu dem entsprechenden Amin umzusetzen (Ann.Chim. (Rome) *47*, 410 (1957)) und Chem. Reviews *65*, 53 bis 68 (1965)).

Es wurde ein Verfahren zur Herstellung von Aminohydroxyverbindungen der Formel

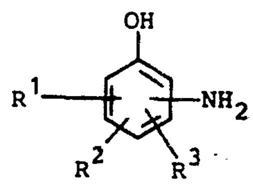

worin

$R^1$, $R^2$ und $R^3$ gleich oder verschieden sind und Wasserstoff, einen neideren Alkylrest, Halogen, eine Carboxyl- oder eine Sulfogruppe oder einen durch Verknüpfung zweier benachbarter Reste gebildeten gegebenenfalls substituierten aromatischen Ring bedeuten,

durch Umsetzung aromatischer Nitrosoverbindungen der Formel

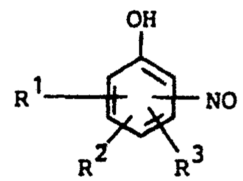

worin

$R^1$, $R^2$ und $R^3$ die obengenannte Bedeutung haben;

mit Hydrazin gefunden, das dadurch gekennzeichnet ist, daß man die aromatische Nitrosoverbindung und 1,0 bis 1,3 Mol Hydrazin je Mol der Nitrosoverbindung in wäßrigem Medium bei Temperaturen von 20 bis 40°C umsetzt, wobei man die aromatische Nitrosoverbindung in Wasser dispergiert und die wäßrige Lösung des Hydrazins nach Maßgabe der Reaktionsgeschwindigkeit eindosiert.

Das erfindungsgemäße Vefahren kann durch die folgende Reaktionsgleichung erläutert werden:

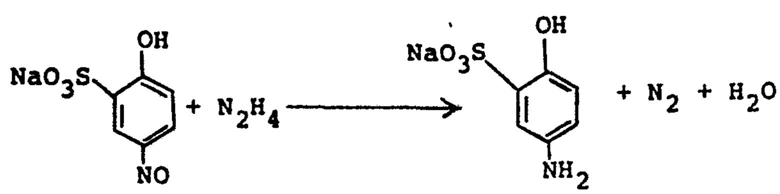

Als niedere Alkylreste seien Kohlenwasserstoffreste mit 1 bis 6 Kohlenstoffatomen, wie Methyl, Äthyl, Propyl, Isopropyl, Butyl, Isobutyl, Pentyl, Isopentyl, Hexyl und Isohexyl, genannt. Bevorzugte niedere Alkylreste sind Methyl und Äthyl.

Als Halogene seien Fluor, Chlor, Brom und Jod, bevorzugt Chlor, genannt.

Als aromatisches Ringsystem, das durch Verknüpfung zweier benachbarter Reste entsteht, sei bevorzugt das Naphthalinsystem ganannt. Dieses kann gegebenenfalls durch einen oder mehrere Reste substituiert sein, wobei die Reste den gleichen Bedeutungsumfang wie $R^1$, $R^2$ und $R^3$ haben.

Als bevorzugte aromatische Nitrosoverbindungen seien Verbindungen der Formel

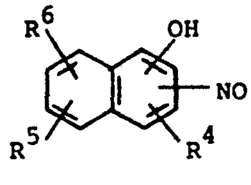

worin

$R^4$, $R^5$ und $R^6$ gleich oder verschieden sind und Wasserstoff oder eine Carboxyl- oder eine Sulfogruppe bedeuten,

genannt.

Die Herstellung der aromatischen Nitrosoverbindungen ist bekannt (Houben-Weyl, Methoden der organischen Chemie, Band X/1, S. 1028—1037 (1971)) und kann beispielsweise durch Umsetzung aromatischer Hydroxyverbindungen mit salpetriger Säure und ihren Derivaten erfolgen.

Beispielsweise seien die folgenden aromatischen Nitrosoverbindungen genannt:

1 - Nitroso - 2 - hydroxy - naphthalin - 4 - solfonsäure, 1 - Nitroso - 2 - hydroxy - naphthalin - 6 - sulfonsäure, 2 - Nitroso - 1 - hydroxy - naphthalin - 4 - sulfonsäure, 1 - Nitroso - 2 - hydroxy - naphthalin - 3,6 - disulfonsäure, 1 - Nitroso - 2 - hydroxy - naphthalin, 1 - Nitroso - 4 - hydroxy - naphthalin, 2 - Nitroso - 1 - hydroxy - naphthalin, 1 - Nitroso - 2 - hydroxy - benzol, 1 - Nitroso - 4 - hydroxy - benzol, 1 - Nitroso - 2 - hydroxy - benzol - 5 - sulfonsäure, 1 - Nitroso - 4 - hydroxy - benzol - 3 - sulfonsäure und 1 - Nitroso - 4 - hydroxy - benzol - 3 - carbonsäure.

Als Hydrazin für das erfindungsgemäße Verfahren kann selbstverständlich auch Hydrazinhydrat eingesetzt werden. Das Hydrazin kann in konzentrierter und in verdünnter wäßriger Lösung eingesetzt werden. Im allgemeinen verwendet man eine wäßrige Hydrazinlösung mit einem Gehalt von 10 bis 90 Gew.-%, bevorzugt von 20 bis 80 Gew.-%, Hydrazin.

Die Nitrosoverbindung und das Hydrazin werden in etwa äquivalenten Mengen eingesetzt. Nach dem erfindungsgemäßen Verfahren setzt man 1,0 bis 1,3 Molteile, bevorzugt 1,05 bis 1,20 Molteile, Hydrazin mit einem Molteil der aromatischen Nitrosoverbindung um.

Das erfindungsgemäße Verfahren wird im Temperaturbereich von 20 bis 40°C, durchgeführt.

Das erfindungsgemäße Verfahren kann bei Normaldruck, erhöhtem oder vermindertem Druck durchgerführt werden. Im allgemeinen wird es bei Atmosphärendruck durchgeführt.

Das erfindungsgemäße Verfahren wird in wäßrigem Medium durchgeführt. Es ist aber selbstverständlich möglich, daß noch weitere Lösungs- oder Verdünnungsmittel wie Alkohole, z.B. Methanol oder Äthanol, oder cyclische Äther, z.B. Dioxan, verwendet werden. Bevorzugtes Lösungsmittel für das erfindungsgemäße Verfahren ist Wasser.

Das erfindungsgemäße Verfahren wird im allgemeinen im pH-Bereich von 4 bis 14, vorzugsweise von 8 bis 12, durchgeführt. Gegebenenfalls können stärker saurere Reaktionsmischungen durch Zugabe von Basen, z.B. Natronlauge, vor der Umsetzung mit Hydrazin auf einen pH-Wert von 4 bis 14 eingestellt werden.

Das erfindungsgemäße Verfahren kann beispielsweise wie folgt durchgeführt werden:

Die Nitrosoverbindung wird in Wasser dispergiert und die wäßrige Lösung des Hydrazins nach Maßgabe der Reaktionsgeschwindigkeit eindosiert. Nach Beendigung der Umsetzung kann das Reaktionsprodukt in üblicher Weise, beispielsweise durch Ansäuren mit Salzsäure, ausgefällt und dann isoliert werden. Falls erforderlich, sind weitere Reinigungsmaßnahmen wie Umkristallisieren oder Umfällen möglich. Durch reguilierung der Zutropfgeschwindigkeit des Hydrazins kann die Reaktionsgeschwindigkeit leicht gesteuert werden. Da sie abhängig von den apparativen Parametern ist, wird der optimale Wert Zweckmäßigerweise für jede Reaktionsapparatur ermittelt. Im allgemeinen kann man das Hydrazin mit einer Geschwindigkeit von 1 bis 10 [ml/Minute] zu der aromatischen Nitrosoverbindung tropfen.

In einer bevorzugten Ausführungsform des erfindungsgemäßen Verfahrens wird die Nitrosoverbindung in Wasser gelöst bzw. suspendiert und nach Einstellen eines pH-Wertes auf 8 bis 12 und einer Innentemperatur von etwa 30°C eine 20- bis 40 %ige wäßrige Lösung von Hydrazinhydrat nach Maßgabe der Reaktionsgeschwindigkeit eindosiert. Die Temperatur wird im Bereich von 20 bis 40°C gehalten. Der Reaktionsablauf kann durch die Gasentwicklung leicht kontrolliert werden.

Das erfindungsgemäße Verfahren läßt sich vorteilhafterweise in einfacher Weise bei geringem apparativen Aufwand durchführen. Vorteilhafterweise kann man das erfindungsgemäße Verfahren in wäßriger Lösung bzw. Suspension durchführen, so daß eine Belastung durch andere Lösungsmittel nicht erfolgt.

Vorteilhafterweise läßt sich das erfindungsgemäße Verfahren ohne Zusatz von Metallkatalysatoren durchführen, die bei Hydrierungen mit Hydrazin oft erforderlich sind.

Da bekannt ist (Houben-Weyl, Methoden der organischen Chemie, Band XI/1, S. 452—457, S. 490—494 (1957)) daß Nitrosoverbindungen mit Hydrazin entweder bei höheren Temperaturen ohne Katalysator oder unter milden Bedingungen mit Hilfe eines Katalysators durchgeführt werden können, ist das erfindungsgemäße Verfahren auch überraschend.

Die nach dem erfindungsgemäßen Verfahren herstellbaren aromatischen Aminohydroxyverbindungen können als fotografische Enwickler oder als Zwischenprodukte für Azofarbstoffe eingesetzt werden.

Beispiel 1

In eine auf 30°C erwärmte Suspension von 388 g (1,0 Mol) 1 - Nitroso - 2 - hydroxy - naphthalin - 6 - sulfonsäure (Na-Salz; 65,3 %ig) in einem Liter Wasser werden unter Rühren 235 ml

(1,1 Mol) einer 23,5 %igen wäßrigen Lösung von Hydrazinhydrat innerhalb von 60 Minuten bei einem pH-Wert von 8 bis 12 eingetropft, wobei die Innentemperatur bei 25 bis 35°C gehalten wird. Die Mischung wird noch etwa eine Stunde bei der angegebenen Temperatur gerührt, bis die Gasentwicklung praktisch beendet ist. Durch Zutropfen von 75 ml 18%iger Salzsäure wird der pH-Wert auf ungefähr 1 eingestellt und 1 - Amino - 2 - hydroxy - naphthalin - 6 - sulfonsäure als Betain in Form rosastichiger Kristalle ausgefällt. Nach Kühlen mit Eis und kurzem Rühren wird abgesaugt, mit Methanol gewaschen und bei 40°C im Vakuum getrocknet.

Ausbeute: 210 g (88% bzw. 82% der Theorie unter Berücksichtigung des cerimetrisch bestimmten Gehalts von 93,3%).

| | | | | |
|---|---|---|---|---|
| Ber.: | C 50,2 | H 3,8 | N 5,9 | S 13,4 |
| Gef.: | C 50,4 | H 4,0 | N 6,3 | S 13,2 |

**Patentanspruch**

Verfahren zur Herstellung von Aminohydroxyverbindungen der Formel

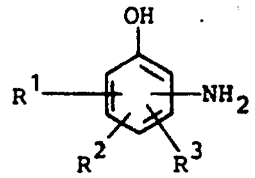

worin

$R^1$, $R^2$ und $R^3$ gleich oder verschieden sind und Wasserstoff, einen niederen Alkylrest, Halogen, eine Carboxyl- oder eine Sulfogruppe oder einen durch Verknüpfung zweier benachbarter Reste gebildeten gegebenenfalls substituierten aromatischen Ring bedeuten,

durch Umsetzung aromatischer Nitrosoverbindungen der Formel

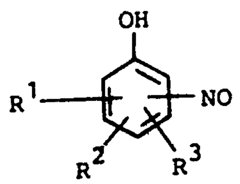

worin

$R^1$, $R^2$ und $R^3$ die obengenannte Bedeutung haben;

mit Hydrazin, dadurch gekennzeichnet, daß man die aromatische Nitrosoverbindung und 1,0 bis 1,3 Mol Hydrazin je Mol der Nitrosoverbindung in wäßrigem Medium bei Temperaturen von 20 bis 40°C umsetzt, wobei man die aromatische Nitrosoverbindung in Wasser dispergiert und die wäßrige Lösung des Hydrazins nach Maßgabe der Reaktionsgeschwindigkeit eindosiert.

**Claim**

Process for the preparation of aminohydroxy compounds of the formula

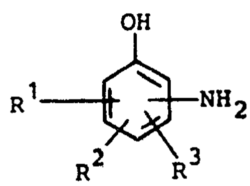

wherein

$R^1$, $R^2$ and $R^3$ are identical or different and denote hydrogen, a lower alkyl radical, halogen, a carboxyl group or a sulpho group or an optionally substituted aromatic ring formed by linking two adjacent radicals,

by reacting aromatic nitroso compounds of the formula

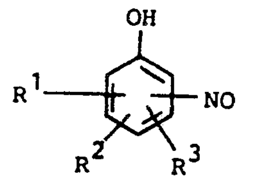

wherein
$R^1$, $R^2$ and $R^3$ have the above-mentioned meaning;
with hydrazine, characterised in that the aromatic nitroso compound and 1.0 to 1.3 mols of hydrazine per mol of the nitroso compound are reacted in an aqueous medium at temperatures from 20°C to 40°C, the aromatic nitroso compound being dispersed in water and the aqueous solution of the hydrazine being introduced at a rate corresponding to the rate of reaction.

**Revendication**

Procédé pour la préparation de composés aminohydroxylés répondant à la formule

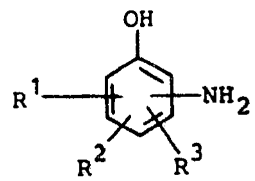

dans laquelle
$R^1$, $R^2$ et $R^3$, identiques ou différents, représentent l'hydrogène, un reste alkyle inférieur, un halogène, un groupe carboxyle ou sulfo, ou un cycle aromatique éventuellement substitué formé par liaison de deux restes voisins,
par réaction de composés aromatiques nitrosés répondant à la formule

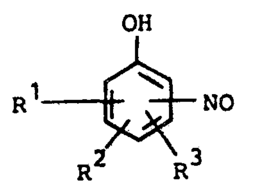

dans laquelle
$R^1$, $R^2$ et $R^3$ ont les significations indiquées ci-dessus,
avec l'hydrazine, ce procédé se caractérisant en ce l'on fait réagir le composé aromatique nitrosé et 1,0 à 1,3 mole d'hydrazine par mole du composé nitrosé en milieu aqueux, à des températures de 20 à 40°C, en dispersant le composé aromatique nitrosé dans l'eau et en ajoutant la solution aqueuse d'hydrazine en proportion de la vitesse de réaction.